# EUROPÄISCHE PATENTANMELDUNG

(11) **EP 1 354 611 A2**
(43) Veröffentlichungstag der Anmeldung: **22.10.2003**
(21) Anmeldenummer: 03007025.4
(22) Anmeldetag: 27.03.2003
(51) Int. Cl.: A61N 2/02

(54) **Vorrichtung zur körpernahen Erzeugung eines natürlichen elektromagnetischen Wechselfelds**

(30) Priorität: 17.04.2002 DE 10217083
(71) Anmelder: Konig, Florian Meinhard, D-82110 Germering (DE)
(72) Erfinder: Konig, Florian Meinhard, D-82110 Germering (DE)
(74) Vertreter: Englaender, Klaus, Dipl.-Ing.

(57) **Zusammenfassung**

Die Erfindung betrifft eine Vorrichtung zur körpernahen Erzeugung eines natürlichen elektromagnetischen Wechselfelds, wie etwa eines Schönwetterfelds (Sferics), insbesondere zur Kompensation von auf einen Nutzer einwirkendem Elektrostress (Technics) und/oder zur positiven Stimulation des Befindens eines Nutzers, mit einem Mittel (10, 13) zum Erzeugen des Wechselfelds und mit einem Mittel (16) zum Aussenden des Wechselfelds.

## Beschreibung

Die Erfindung betrifft eine Vorrichtung zur körpernahen Erzeugung eines natürlichen elektromagnetischen Wechselfelds.

Unsere Umwelt ist durch zahlreiche elektromagnetische Wechselfelder belastet. Ein großer Teil dieser elektromagnetischen Wechselfelder ist künstlichen, d.h. vom Menschen erzeugten Ursprungs. Ein aktuelles prominentes Beispiel für derartige Felder sind künstlich erzeugte elektromagnetische Felder zur Datenübertragung bis in den Giga-Hertz-Bereich. Ein Beispiel hierfür ist das Mobil-Telefon, das sogenannte Handy, das im GSM- und zukünftig auch im UMT-Datenübertragungsformat arbeitet. Diese elektromagnetischen Felder besitzen typischerweise eine Pulsung im Bereich von 100 Hz bzw. 217 Hz bei Handys. Hiermit verbunden ist ein starker Elektro-Stress, der sich auf organische Zellen ungünstig auswirkt. Insbesondere bei Handys ist aufgrund der hohen Sendeleistung dieser Geräte unter Nutzung des jeweiligen Handgeräts am Ohr eines Nutzers eine deutliche biologische Stresseinwirkung auf organische Zellen des Nutzers nicht vermeidbar. In wissenschaftlichen Publikationen und unter anderem im Magazin 2000 plus, Nr. 144 (12-1999, S. 74), wurde von Liebrecht von Klitzing ausgeführt, dass die hohe Dynamik von über 60 dB zwischen den Pulspaketen der HF-Signalübertragung Auslöser für heftige Zellreizungen/Irritationen bei einer typischen Zellresonanz von bis zu 400 Hz ist. Ferner ist in diesem Artikel ausgeführt, dass die Signalperiodizität einen wesentlichen Faktor bei der Zellbeeinflussung darstellt, und dass ein stochastisches Signalverhalten für die organischen Zellen wesentlich günstiger, weil unschädlicher ist. Darüber hinaus sind über ungünstige biologische Einwirkungen von künstlichen Hochfrequenzfeldern auf organische Zellen von Lebewesen, insbesondere Menschen, hinreichend Kenntnisse verfügbar (siehe beispielsweise Herbert L. König "unsichtbare Umwelt", München, 1986).

Belastung des menschlichen Körpers liegt aber auch in Form von natürlichen elektromagnetischen Wechselfeldern vor. So ist bekannt, dass bestimmte Wetterlagen, beispielsweise Fönlagen im Alpenbereich, mit natürlichen elektromagnetischen Wechselfeldern verbunden sind, die sich horizontal und/oder vertikal ausbreiten und im Bereich zwischen einigen 100 Hz bis typischerweise 50.000 Hz liegen. Derartige "Schlechtwetterfelder" wirken sich in bekannter Weise (siehe beispielsweise Fön) ungünstig auf Lebewesen aus. Im Gegensatz hierzu ist von Schönwetterfeldern, auch als Sferics bezeichnet, bekannt, dass sie eine positive Auswirkung auf Lebewesen haben. Auch diese natürlichen elektromagnetischen Wechselfelder liegen im Bereich von einigen 100 Hz bis typischerweise zumindest 20 kHz vor und zeigen im Gegensatz zu den Schlechtwetterfeldern eine andere Amplituden- und Frequenzverteilung.

Ferner ist bekannt, zur Stimulation des menschlichen Organismus und zur Kompensation bestimmter ungünstiger Einwirkungen auf diesen sowie zur Beruhigung und allgemein zur Kompensation von Elektrosmog ein batteriebetriebenes Taschengerät einzusetzen, das künstliche elektromagnetische Wechselfelder im Niedrigstfrequenzbereich zwischen 1,4 und 32,4 Hz erzeugt ("MEDISENT", www.magnet-feldtherapie-2000.de). Höherfrequente elektromagnetische Wechselfelder, wie etwa Elektrosmog im NF-Bereich oberhalb von 30 Hz und im HF-Bereich werden durch dieses Gerät nicht berücksichtigt.

Eine Aufgabe der vorliegenden Erfindung besteht deshalb darin, eine Vorrichtung zu schaffen, durch die auf einen Nutzer einwirkender Elektro-Stress zumindest im wesentlichen unabhängig von Amplitude und Frequenz kompensiert und/oder das Befinden eines Nutzers positiv stimuliert werden kann.

Gelöst wird diese Aufgabe durch die Merkmale des Anspruchs 1. Vorteilhafte Weiterbildungen der Erfindung sind in den Unteransprüchen angegeben.

Demnach schafft die vorliegende Erfindung eine Vorrichtung zur körpernahen Erzeugung eines natürlichen elektromagnetischen Wechselfelds, wie etwa eines Schönwetterfelds (Sferics), insbesondere zur Kompensation von auf einen Nutzer einwirkendem Elektrostress (Technics) und/oder zur positiven Stimulation des Befindens eines Nutzers, mit einem Mittel zum Erzeugen des Wechselfelds und mit einem Mittel zum Aussenden des Wechselfelds.

Mit anderen Worten basiert die Erfindung auf der Erkenntnis, dass sowohl natürliche wie künstliche elektromagnetische Wechselfelder, die sich ungünstig auf Lebewesen (folgend auch als Nutzer bezeichnet) auswirken, durch die Erzeugung eines Gegenfelds, nämlich eines natürlichen elektromagnetischen Wechselfelds, dessen positive Einwirkungen auf einen Nutzer bekannt sind, kompensiert werden können. Alternativ oder ergänzend hierzu kann ein derartiges Gegenfeld auch zur positiven Stimulation des Befindens eines Nutzers beitragen. Besonders geeignet als Gegenfeld haben sich Schönwetterfelder (Sferics) erwiesen.

Erfindungsgemäß wird das Gegenfeld in Gestalt des natürlichen elektromagnetischen Wechselfelds körpernahe erzeugt. Als körpernahe wird vorliegend die Quelle eines elektromagnetischen Wechselfelds bezeichnet, die unmittelbar am Körper, beispielsweise im Bereich eines Bekleidungsstücks eines Nutzers liegt, sowie im Nahfeld eines Nutzers, d.h., in der unmittelbaren Umgebung, in der er sich aufhält, wie beispielsweise der Wohnbereich, eine Kraftfahrzeug-Fahrgastzelle und ein Gebäude. Begrenzt ist dieser Nahbereich allgemein durch den Fernbereich, der definiert ist durch den Bereich, der an das unmittelbare Umfeld eines Nutzers angrenzt, wie beispielsweise urbane Einrichtungen einer Stadt außerhalb von dem Raum bzw. dem Gebäude, in dem sich ein Nutzer aufhält.

Die erfindungsgemäße Vorrichtung zur körpernahen Erzeugung eines natürlichen elektromagnetischen Wechselfelds umfasst, ein Wechselfelderzeugungsmittel und ein Wechselfeldaussendemittel. Vorteilhafterweise sind diese beiden Mittel in einem gemeinsamen Gehäuse aufgenommen, das im Nahfeld eines Nutzers positioniert und gegebenenfalls unmittelbar am Körper eines Nutzers getragen wird, beispielsweise an einem Bekleidungsstück. Im zuletzt genannten Fall kann das Gehäuse beispielsweise in Art eines Kugelschreibers oder alternativ in Gestalt eines flachen Behälters gebildet sein. Wenn besagte Einheit im Nahbereich eines Nutzers, jedoch nicht am Körper getragen zum Einsatz kommt, kann sie beispielsweise in einem Möbelstück, vor allem ein Sitzmöbel integriert sein, oder aber in dem Bereich der Fahrgastzelle eines Kraftfahrzeugs oder eines sonstigen Fahrzeugs, beispielsweise auch eines Luftfahrzeugs oder eines Wasserfahrzeugs.

Die erfindungsgemäße Vorrichtung kann aber auch in einem Gerät enthalten sein, das künstliche elektromagnetische Felder erzeugt, die durch die erfindungsgemäße Vorrichtung kompensiert werden sollen, beispielsweise in einem Handy oder einem schnurlosen Telefon, einem Kopfhörer, in Komponenten einer Audio-/Videoanlage und dergleichen.

Gemäß einer besonders vorteilhaften Weiterbildung der Erfindung umfasst das Mittel zum Aussenden des erfindungsgemäßen natürlichen elektromagnetischen Wechselfelds bereits in der Umgebung eines Nutzers vorhandene "Sendeantennen", wie etwa die Schutzkontakterdung eines Gebäudes, Verbindungsleitungen eines Telekommunikationsanschlusses, aber auch einen Faraday'schen Käfig, wie etwa die Karosserie eines Fahrzeugs, wie etwa eines Kraftfahrzeugs, eines Flugzeugs, oder eines Schiffs, oder sonstige Sendemittel innerhalb dieser Umgebungen.

Vorteilhafterweise umfasst das Mittel zur Erzeugung des natürlichen elektromagnetischen Wechselfelds einen Speicher, in welchem dieses Wechselfeld bevorzugt in digitaler Form abgelegt ist. Alternativ sind in digitaler Form mehrere Wechselfelder im Speicher abgelegt und zum Anlegen an das Sendemittel aus diesem bevorzugt tageszeitabhängig abrufbar. Bevorzugt sind in dem Speicher die Daten bezüglich eines Schönwetterfelds (Sferics) abgespeichert, wobei die Daten für dieses Schönwetterfeld vor Ort erfasst, daraufhin in digitale Form umgesetzt und im Speicher abgelegt werden.

Je nach Bedarf können dem erfindungsgemäß genutzten natürlichen elektromagnetischen Wechselfeld auch künstliche Wechselfelder zugemischt werden, die gegebenenfalls ebenfalls in einem Speicher in digitaler Form abgelegt sind, und von denen bekannt ist, dass sie die Kompensation von beispielsweise Elektro-Stress unterstützen und/oder zur positiven Stimulation des Befindens eines Nutzers beitragen. Zu nennen ist hier als künstliches elektromagnetisches Wechselfeld z.B. ein niedrigstfrequentes Signal, wie etwa ein Breitbandimpuls im Frequenzbereich von 0,01 bis 40 Hz, bevorzugt 0,1 bis 32 Hertz (Niedrigstfrequenzbereich).

Die Erfindung wird nachfolgend anhand der Zeichnung beispielhaft näher erläutert; in dieser zeigen:
- Fig. 1: ein Blockschaltbild einer erfindungsgemäßen Vorrichtung,
- Fig. 2: einen Auszug eines typischen Schönwetterfelds (frequenzbereichseingegrenzte Sferics-Darstellung), das in digitaler Form in der Vorrichtung von Fig. 1 abgespeichert ist,
- Fig. 3: in Draufsicht eine Schallwand in der Muschel eines Kopfhörers, und
- Fig. 4: eine Längsschnittansicht der Schallwand von Fig. 3 entlang deren Längsmittenachse mit einer erfindungsgemäßen Vorrichtung.

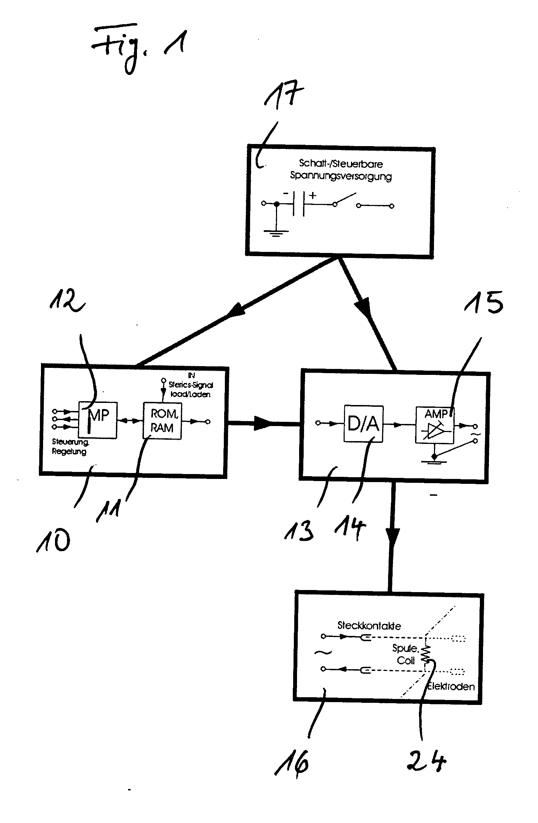

Wie aus dem Blockschaltdiagramm von Fig. 1 hervorgeht, umfasst die erfindungsgemäße Vorrichtung zur körpemahen Erzeugung eines natürlichen elektromagnetischen Wechselfelds eine Speichereinrichtung 10, in der das elektromagnetische Wechselfeld bzw. mehrere derartige Wechselfelder in digitaler Form gespeichert sind. In der in Fig. 1 dargestellten Ausführungsform enthält die Speichereinrichtung 10 einen ROM bzw. RAM 11 und eine Steuerschaltung 12, mit der die Daten eines jeweiligen elektromagnetischen Wechselfelds in dem ROM bzw. RAM 11 aufgerufen und gezielt am Ausgang hiervon bereitgestellt werden können. Der Ausgang der Speichereinrichtung 10 ist mit einer D/A-Wandlereinheit 13 verbunden, in der die am Ausgang der Speichereinrichtung 10 anliegenden digitalen Daten in analoge Signale umgesetzt werden. Zu diesem Zweck umfasst die Einrichtung 13 ein D/A-Wandlermodul 14, das eingangsseitig vom Ausgangssignal der Einrichtung 10 beaufschlagt wird, und einen bezüglich seines Verstärkerfaktors einstellbaren Verstärker 15, der sich an den Ausgang des D/A-Wandlermoduls 14 anschließt, und an dessen Ausgang die analogen Signale mit einer bestimmten Amplitude anliegen.

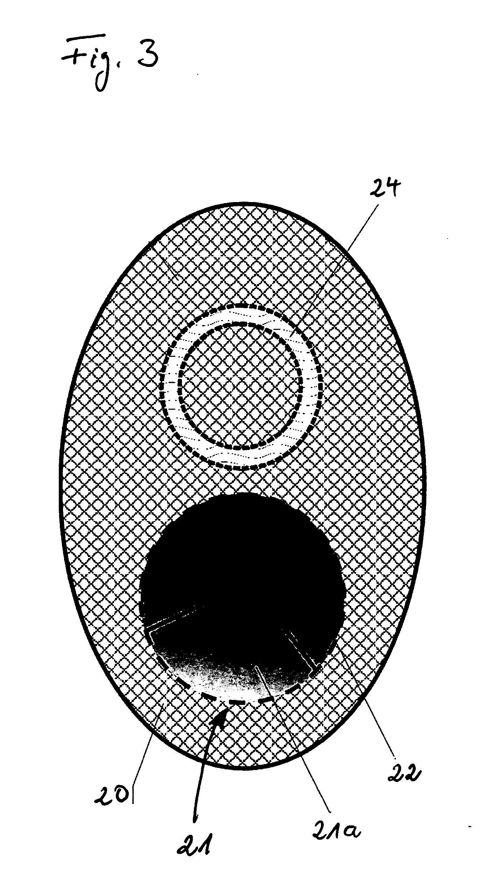

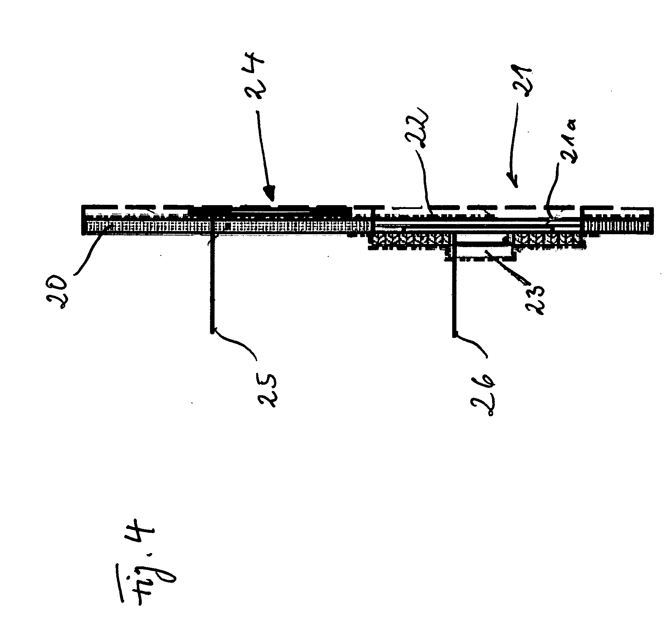

Das Ausgangssignal des Verstärkers 15 bzw. der Wandlereinheit 13 ist mit einer Sendeeinrichtung 15 verbunden, durch die das natürliche elektromagnetische Wechselfeld abgestrahlt wird, damit dieses körpernahe durch einen Nutzer aufgenommen werden kann. Die Sendeeinrichtung 16 kann mit einer Sendeantenne verbunden sein, wie beispielsweise der Schutzkontakt eines Gebäudes oder mit der Karosserie eines Fahrzeugs, um das natürliche elektromagnetische Wechselfeld zu einem Nutzer innerhalb eines Gebäudes oder dem Fahrer oder sonstigen Insassen der Fahrgastzelle eines Fahrzeugs zu übertragen. In Fig. 3 und 4 ist eine Muschel eines Kopfhörers dargestellt, die unhörbar zur Übertragung des natürlichen elektromagnetischen Wechselfelds ausgelegt ist, wie nachfolgend im einzelnen ausgeführt.

Außerdem umfasst die in Fig. 1 gezeigte erfindungsgemäße Vorrichtung ein Netzteil 17 zur Stromversorgung der Einrichtungen 10, 13 und 16.

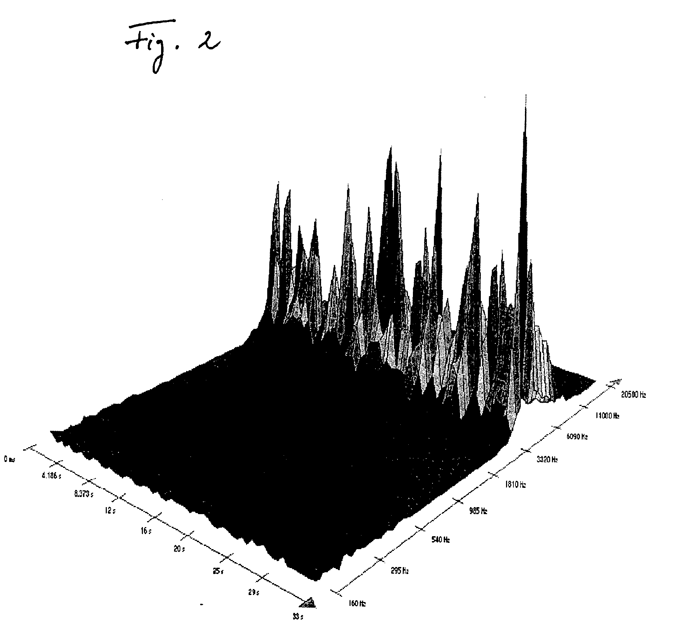

In Fig. 2 ist ein schmalbandiger Auszug bzw. ein frequenzbegrenztes Beispiel eines natürlichen elektromagnetischen Wechselfelds in Gestalt eines Schönwetterfelds (Sferics) gezeigt, das nach seiner Erfassung gesampelt und als eines der Wechselfelder in der Speichereinrichtung 10 abgelegt wird.

Nunmehr wird auf Fig. 3 und 4 Bezug genommen. Dargestellt ist die Schallwand 20 einer Kopfhöhermuschel, deren Aufbau vorliegend nicht weiter von Interesse ist. Die Schallwand 20 ist allgemein ellipsenförmig und in ihrem unteren Bereich ist eine kreisförmiger Durchbruch vorgesehen, in den ein Schallwandler 21 eingesetzt ist, vor welchem zur Förderung eines vorne mittig vor einem Nutzer wahrgenommenen Hörereignisses eine Schallblende 22 angeordnet ist, die die Membran 21a des Schallwandlers 21 teilweise abdeckt.

Wie aus Fig. 4 hervorgeht, wird die Membrane 21a des Schallwandlers 22 in herkömmlicher Weise von einem elektromagnetischen Antrieb angetrieben, der auf der Rückseite der Membran sitzt und mit dieser verbunden ist. Dieser Antrieb umfasst eine Spule 23, die von einem Nutzsignalstrom durchflossen ist und ein elektromagnetisches Wechselfeld (vorliegend als künstliches elektromagnetisches Wechselfeld im Gegensatz zum erfindungsgemäßen Gegenfeld bezeichnet, das ein natürliches elektromagnetisches Wechselfeld ist) erzeugt, das aufgrund seiner Nähe an der Schläfe eines Nutzers für diesen Elektro-Stress hervorruft. Um diesen Elektro-Stress zu kompensieren, ist von der Längsmittenachse der Schallwand 20 durchsetzt eine ringkreisförmige Spule 24 beabstandet von dem Schallwandler 21 vorgesehen. Diese Spule 24, die schematisch auch als Bestandteil der Einrichtung 16 in Fig. 1 gezeigt ist, wird über Anschlüsse 25 und 26 mit dem natürlichen elektromagnetischen Wechselfeld, das am Ausgang des Verstärkers 15 anliegt, beaufschlagt (alternativ zur Spule 24 kommt eine sonstige "Antenne" zur Abstrahlung" eines Gegenfelds zum elektromagnetischen Feld des Schallwandlers 21 in Betracht). Es hat sich herausstellt, dass dieses von der Spule 24 erzeugte natürliche elektromagnetische Wechselfeld die ungünstigen, zu Elektro-Stress führenden Wirkungen des künstlichen elektromagnetischen Wechselfelds kompensiert, die von der Spule 23 des Schallwandlerantriebs erzeugt wird. Bevorzugt ist die erfindungsgemäße Vorrichtung 10, 13, 16 in die Kopfhörermuschel(n) integriert.

Bevorzugte Anwendungsbereiche der erfindungsgemäßen Vorrichtung zur körpernahen Erzeugung eines natürlichen elektromagnetischen Wechselfelds sind im folgenden aufgelistet:
1. Am Köroper tragbare Vorrichtungen, vorzugsweise in Gestalt eines Schreibstifts oder eines andere bevorzugt zylindrischen Gegenstands, der tragbar ist beispielsweise in der Bekleidung von Erwachsenen, Kindern sowie nicht menschlichen Lebewesen, wie etwa Hunden und Nutztieren. Im letztgenannten Fall kommt auch ein Einsatz als Halsband in Betracht.
2. Anwendung der Vorrichtung zur indirekten Nutzung durch Lebewesen in Gestalt von Generatoren für Wohnräume, beispielsweise in Form eines Designgegenstands, in welchen die erfindungsgemäße Vorrichtung eingebaut ist, oder in ein Kissen integrierte. Für besonders belastete Räume, beispielsweise Büroräume kommt eine erfindungsgemäße Vorrichtung mit entsprechend großer Sendeleistung in Betracht. Ein weiteres Einsatzgebiet dieser Art betrifft Fahrzeuge, beispielsweise Personenkraftfahrzeuge, mit einer Speisung der erfindungsgemäßen Vorrichtung aus der Fahrzeugbatterie. Einen weiteren Einsatz dieser Art betrifft einen robusten und wasserdicht gestalteten Behälter mit der erfindungsgemäßen Vorrichtung. Robust ausgestaltet in diesem Sinne ist beispielsweise auch eine erfindungsgemäße Vorrichtung, die in Tiergehegen und Ställen zum Einsatz kommt.
   Ein weiteres Einsatzbereich in diesem Gebiet betrifft den Anschluss der erfindungsgemäßen Vorrichtung an elektrisch leitende Elemente, beispielsweise Magnetfeldgeräte. In diesem Zusammenhang kommt auch ein Einsatz der erfindungsgemäßen Vorrichtung in Wellnessgeräten für den Heimeinsatz, für den Einsatz in Hotels, in Fitnessstudios, Arztpraxen, Krankenhäusern usw. in Betracht. Nicht zuletzt ist erfindungsgemäß die Vorrichtung auch als quasi Ionisator für Wasser, beispielsweise Wasserfüllungen von Badewannen und/oder Schwimmbecken möglich.
3. Einsatz der erfindungsgemäßen Vorrichtung im Zusammenhang mit Gegenständen des täglichen Lebens. Zu nennen ist hier eine Integration der erfindungsgemäßen Vorrichtung in Elektrogeräten, die typischerweise unter Stromzuführung ein elektromagnetisches Feld erzeugen, das durch die erfindungsgemäße Vorrichtung bezüglich der Schädlichkeit auf Lebewesen neutralisiert werden soll. Zu nennen sind hierbei Fernsehgeräte, HiFi-Anlagen, Computereinrichtungen, inklusive Monitor, Drucker, Laptop, Notebook. Weitere Geräte dieser Art sind Telefone, inklusive Mobiltelefone. In Betracht kommt in diesem Zusammenhang außerdem der Bereich von Küchengeräten mit Mikrowellen, Herden, Öfen, Spülmaschinen, Herden, Kühlschränken und dergleichen. Besonders wirksam lässt sich die erfindungsgemäße Vorrichtung auch im Zusammenhang mit Halogentransformatoren bzw. Niedervolttransformatoren einsetzen. Im Zusammenhang mit Schlaflichtern, Lampen/Leuchten und Salzkristallen. In diesen Bereich fällt auch der Einsatz der erfindungsgemäßen Vorrichtung in Kraftfahrzeugen, Flugzeugen, Zügen, im Schlafbereich von Lebewesen, direkt in der Kleidung derselben, in Schmuckstücken und dergleichen.
4. Einsatz der erfindungsgemäßen Vorrichtung im Zusammenhang mit der medizinischen Therapie zur Bekämpfung von u.a. Migräne, Schlafstörungen, Erschöpfungszuständen, Asthma, Stresssymptomen und dergleichen.

## Patentansprüche

1. Vorrichtung zur körpernahen Erzeugung eines natürlichen elektromagnetischen Wechselfelds, wie etwa eines Schönwetterfelds (Sferics), insbesondere zur Kompensation von auf einen Nutzer einwirkendem Elektrostress (Technics) und/oder zur positiven Stimulation des Befindens eines Nutzers, mit einem Mittel (10, 13) zum Erzeugen des Wechselfelds und mit einem Mittel (16) zum Aussenden des Wechselfelds.

2. Vorrichtung nach Anspruch 1, **dadurch gekennzeichnet, dass** die Einheit aus Erzeugungsmittel (10, 13) und Sendemittel (16) in einem Gehäuse zum körpernahen Positionieren der Anordnung aufgenommen ist.

3. Vorrichtung nach Anspruch 2, **dadurch gekennzeichnet, dass** das Gehäuse zur Aufnahme in und/oder zum Tragen an einem Bekleidungsstück eines Nutzers ausgelegt ist.

4. Vorrichtung nach Anspruch 1, **dadurch gekennzeichnet, dass** die Einheit aus Erzeugungsmittel und Sendemittel im Umfeld eines Nutzers, vor allem in bzw. an einem körpernah genutzten Gegenstand angeordnet ist.

5. Vorrichtung nach Anspruch 4, **dadurch gekennzeichnet, dass** das Umfeld ein Wohnraum und der Gegenstand ein Möbelstück, vor allem ein Sitzmöbel ist.

6. Vorrichtung nach Anspruch 4, **dadurch gekennzeichnet, dass** das Umfeld die Fahrgastzelle eines Fahrzeugs, z.B. eines Kraftfahrzeugs, eines Flugzeugs, eines Schiffes und der Gegenstand vor allem ein Fahrzeugsitz ist.

7. Vorrichtung nach Anspruch 1, **dadurch gekennzeichnet, dass** die Einheit aus Erzeugungsmittel (10, 13) und Sendemittel (16) in einem elektrische bzw. elektromagnetische Felder erzeugenden Einrichtung (20) enthalten und insbesondere zur Kompensation bzw. Ergänzung dieser Felder ausgelegt ist.

8. Vorrichtung nach Anspruch 7, **dadurch gekennzeichnet, dass** die Einrichtung (10, 13)aus der Gruppe ausgewählt ist, die Kopfhörer, Komponenten einer Audio/Videoanlage, Handys, drahtlose Telefone, Computer und dgl. umfasst.

9. Vorrichtung nach einem der Ansprüche 1 bis 8, **dadurch gekennzeichnet, dass** das Mittel (10, 13) zur Erzeugung des natürlichen elektromagnetischen Wechselfelds einen Speicher (11) umfasst, in welchem dieses Wechselfeld in digitaler Form abgelegt ist.

10. Vorrichtung nach Anspruch 9, **dadurch gekennzeichnet, dass** im Speicher (11) verschiedene Wechselfelder angelegt und aus diesem durch eine Wahleinrichtung (12) abrufbar sind.

11. Vorrichtung nach Anspruch 9 oder 10, **dadurch gekennzeichnet, dass** ein Mittel zum Erzeugen eines vom natürlichen elektromagnetischen Wechselfeld unterschiedlichen künstlichen elektromagnetischen Wechselfelds, z.B. eines niedrigstfrequenten Signals, wie etwa einen unipolaren oder bipolaren Breitbandimpuls im Frequenzbereich von 0,01 bis 40 Hz und/oder eines Signals im ELF-Bereich vorgesehen ist, wobei dieses künstliche Wechselfeld mit dem natürlichen Wechselfeld gemischt und gemeinsam mit diesem in das Sendemittel (16) eingespeist wird.

12. Vorrichtung nach einem der Ansprüche 1 bis 11, **dadurch gekennzeichnet, dass** das natürliche elektromagnetische Wechselfeld Sferics in Gestalt mindestens eines gesampelten Schönwetterfelds umfasst.

13. Vorrichtung nach einem der Ansprüche 1 bis 5, bzw. 9 bis 12, **dadurch gekennzeichnet, dass** das natürliche elektromagnetische Wechselfeld zur Einwirkung auf einen Nutzer in eine die Schutzkontakterdung eines Gebäudes oder dgl. eingespeist ist.

14. Vorrichtung nach einem der Ansprüche 1 bis 5, bzw. 9 bis 12, **dadurch gekennzeichnet, dass** das natürliche elektromagnetische Wechselfeld zur Einwirkung auf einen Nutzer-in die Verbindungsleitungen eines Telekommunikationsanschlusses, die Erdungs-SCHUKO-Leitung der Hausstrom-Versorgung oder dgl. eingespeist ist.

15. Vorrichtung nach einem der Ansprüche 1 bis 5, bzw. 9 bis 12, **dadurch gekennzeichnet, dass** das natürliche elektromagnetische Wechselfeld zur Einwirkung auf einen Nutzer in die Karosserie eines Fahrzeugs oder dgl. eingespeist ist.

16. Vorrichtung nach einem der Ansprüche 1 bis 15, **dadurch gekennzeichnet, dass** die Erzeugungsmittel (10, 13) bzw. die Sendemittel (16) dazu ausgelegt sind, das natürliche elektromagnetische Wechselfeld periodisch, vorzugsweise fadened (Anstiegs/Abfallsflanke bei wechselndem Ein/Aus-Betrieb) bereitzustellen.

17. Vorrichtung nach einem der Ansprüche 1 bis 16, **dadurch gekennzeichnet, dass** die Erzeugungsmittel (10, 13) bzw. die Sendemittel (16) dazu ausgelegt sind, das natürliche elektromagnetische Wechselfeld einem Tagesgang entsprechend in unterschiedlicher Gestalt bereitzustellen.
